# EUROPEAN PATENT APPLICATION

(11) **EP 1 420 254 A2**
(43) Date of publication of application: **19.05.2004**
(21) Application number: 03024739.9
(22) Date of filing: 29.10.2003
(51) Int. Cl.: G01N 33/68

(54) **Amyloid quantification**

(30) Priority: 06.11.2002 EP 02024924
(71) Applicant: F. HOFFMANN-LA ROCHE AG, 4070 Basel (CH)
(72) Inventor: Bohrmann, Bernd, 79100 Freiburg (DE); Doebeli, Heinz, 4417 Ziefen (CH); Ducret, Axel, 4125 Riehen (CH); Guentert, Andreas, 4461 Boeckten (CH)

(57) **Abstract**

The present invention relates to an *in vitro* method for the quantification of beta-amyloid peptide (Aβ) in mammalian tissue samples and body fluids comprising spiking of isotope labeled Aβ into a sample containing Aβ and determining labeled and unlabeled Aβ by mass spectrometry. The present invention also relates to the use of the methods of the invention for the determination of the Aβ content in tissue sample and body fluid as well as the determination of Aβ microheterogeneities.

## Description

The present invention relates to an in vitro method for the quantification of beta-amyloid peptide (Aβ) in mammalian tissue samples and body fluids comprising isotope dilution and mass spectrometry.

Due to the dramatic rise in life expectancy during the 20^{th} century from approximately 49 years to more than 76 years, an increasing number of individuals is reaching the age in which neurodegenerative disorders become common. Among these, the Alzheimer's disease (AD), which was first described by Alois Alzheimer 1906, has emerged to the most prevalent form of late-life mental failure in humans.

Several cardinal features can be observed in most patients: progressive memory impairment, disordered cognitive function, altered behavior and a progressive decline in language function. The process of neurodegeneration and the pathological changes linked to AD are subject to intense research. However, the molecular mechanism underlying AD is not known yet. Originally described were the dense (neuritic) plaques and the neurofibrillary tangles and therefore they serve(d) many years as post-mortem diagnostic markers for AD.

The diagnostic lesions in the brain of AD patients can be summarized as follows (Selkoe D.J., (2001), Alzheimer's Disease: Genes, Proteins, and Therapy, Physiological reviews 81: 741-766). Neuritic plaques contain extracellular deposits of amyloid β-protein (Aβ), which occurs predominantly in a filamentous form. These extracellular deposits are star-shaped masses of amyloid fibrils, which are surrounded and penetrated by dystrophic neurites. These neurites show ultrastructural abnormalities like enlarged lysosomes, numerous mitochondria and (sometimes) paired helical filaments (aggregated forms of tau-protein). Closely associated with these plaques are microglia and reactive astrocytes. The microglia usually can be found within or adjacent to the central amyloid core, whereas astrocytes often occur in a ring outside of the plaques. This is indicative for an immunoreactive response of the brain. Aβ fibrils have the capacity to fold into what are called "beta-pleated" sheet fibrils, and can experimentally be stained with intercalating agents, such as Congo Red or Thioflavine S. A cross-section of a neuritic plaque reveals a diameter between 10 and 120 µm.

Many different variants of Aβ are known to occur with either heterogeneity at the amino- as well as carboxy-terminus. Of particular interest is the heterogeneity at the carboxy-terminus, since the longer form with 42 amino-acids (Aβ1-42) is much more prone to aggregation than the shorter form containing 40 amino acids (Aβ1-40). Inherited forms of familial Alzheimer's which is characterised by an early onset of the disease strongly suggest the detrimental role of Aβ1-42 in the pathogenesis. Few information concerning the correlation of the heterogeneity of the amino-terminus with the pathogenesis is available, mainly due to the lack of antibodies specific for the amino-terminus.

Despite a world wide research effort there is neither a cure for the disease nor a convenient *pre-mortem* diagnosis. A reliable *pre-mortem* diagnosis is a prerequisite for any clinical trial addressing the disease modifying effect of a drug. Disease markers could be the amyloid peptide or derivatives thereof taken from serum, CSF or as a biopsy from brain. In addition, a method which unambiguously allows to compare human brain specimen with specimens from transgenic animals would be important to proof the validity of any drug trial performed with these animals.

However, several properties of Aβ render its determination difficult. The most obvious property is the aggregation of the peptide and the fact that the fibres have to be disintegrated by harsh procedures. Another property is the stickiness of the peptide to proteins, e.g. serum albumin. Since serum albumin is present in vast quantity it competes for Aβ binding with antibodies used for instance in an ELISA. Therefore, a critical step in the determination of Aβ is the sample preparation. Extraction from dense plaques, diffuse plaques, vessel walls or separating it from the serum albumin requires dedicated and mostly cumbersome procedures and each such procedure may lead to an unknown loss of Aβ. Thus, methods to determine the amount of Aβ as well as its microheterogeneity could be important in setting up a diagnostic method.

The present invention therefore solves the problem of quantifying Aβ by providing a method which affords a more accurate beta amyloid quantification as well as the quantification of the different forms of Aβ by combining methods comprising isotope dilution and mass spectrometry.

The present invention provides an analytical method which affords the quantification of beta amyloid peptide in mammalian tissue samples and body fluid. The method of the invention comprises isotope dilution and mass spectrometrical determination of the beta amyloid content of a biological sample, thereby providing more accurate results of the beta amyloid content than methods known in the art, e.g. antibody-based procedures. Moreover, the methods of the invention take account of Aβ microheterogeneities by specifically quantitating the different forms of Aβ.
Figure 1: Flow chart of a method for the determination of the Aβ content of plaques derived from brain sections.
Figure 2: Immunostained brain section from transgenic APP^{swe}/PS2 mice before and after laser dissection microscopy and laser pressure catapulting
Figure 3: Example of mass spectrometry profile with ¹⁴N and ¹⁵N Aβ-fragments 1-16 and 17-28. A) Experiment with 100 plaques from transgenic APP^{sWe}/PS2 mice and 5 pmol internal standard; B) Experiment with 100 plaques and 10 pmol internal standard; C) Experiment with 100 plaques and 25 pmol internal standard.
Figure 4: Comparison of the calculated and experimentally observed ¹⁴N and ¹⁵N Aβ-fragments 1-16
Figure 5: Comparison of the calculated and experimentally observed ¹⁴N and ¹⁵N Aβ-fragments 17-28
Figure 6: Determination of the optimal working range;□ :first experiment; ○: second experiment:Δ third experiment (see Fig. 3); Filled icons: Useful range; Open icons: No result obtained; Area in rectangle: Selected working range.
Figure 7: Validation of the method with Western-blot. Lanes 1-5: one single excised mouse plaque, treated with HCOOH (overnight). Lane 6: Size marker. Lane 7-10: 0.1ng synthetic Aβ 1-42. WO-2 Antibody, exposure time was 2 minutes. The detectable amount of Aβ in a plaque is between 0.05ng and 0.2ng.
Figure 8: A) Quantification of the Aβ content of plaques isolated from a transgenic mouse; MS: mass spectrometry; WB: Western blotting; Number required: numer of analysed plaques; B) Experiment: the amount of Aβ in the excized plaques (2D) is analysed. This amount has to be corrected in order to represent the amount of Aβ present in the spherical plaque.

### Methods

Therefore, the present invention provides a method for the quantification of beta amyloid peptide comprising the steps of:
(a) providing a source of beta amyloid
(b) adding a defined amount of beta amyloid peptide labeled with a stable isotope to the source of (a)
(c) isolating unlabeled and labeled beta amyloid
(d) preparing the isolated beta amyloid for analysis by mass spectrometry
(e) analysing the prepared beta amyloid by mass spectrometry, and
(f) determining the amount of beta amyloid that was present in the source of beta amyloid.

With the described method, the present invention provides for a more accurate quantification of Aβ in mammalian tissue samples and body fluid, also taking into account the amount of different Aβ forms.

In the method of the present invention, any source which contains beta amyloid may be used. Sources of beta amyloid comprise tissue samples, e.g. homogenized brain samples, and body fluid. Preferred sources of beta amyloid are amyloid deposits obtained from tissue samples, serum and CSF. Amyloid deposits obtained from tissue samples comprise dense (neuritic or senile) plaques, diffuse plaques, and amyloid deposits in small arterioles and venules, causing a microvascular angiopathy. The amyloid deposits mainly comprise aggregated beta amyloid, besides minor amounts of other components. Most preferred are amyloid plaques obtained from brain tissue.

Amyloid deposits containing aggregated beta amyloid may be obtained from tissue samples by methods comprising general biochemical protein purification methods and methods for specific excision of structures from tissues comprising laser dissection microscopy. Preferably, amyloid deposits are excised from tissue samples by laser dissection microscopy. Preferably, the amyloid deposits are excised from tissue slices, more preferably, they are excised from brain slices.

The laser dissection microscopy method comprises the steps of cold ablation and laser pressure catapulting (Schütze et al (1998), Identification of expressed genes by laser-mediated manipulation of single cells, Nature Biotechnology 16: 737-742; Simone et al (1998), Laser-capture microdissection: opening the microscopic frontier to molecular analysis, TIG 14: 272-276). Laser dissection microscopy can be used to capture any specific phenotypes or phenotypic tissue changes identifiable by light microscopy. As an example, this technique could help in detecting differences in gene expression between normal cells or tissues and pathological material by separate microdissection and analysis (e.g. by microarray) of the isolated specimen. Qualitative and quantitative analysis of critical changes thus can be performed more easily and with more accuracy compared to the analysis of whole tissues as is necessary without laser dissection. The advantages of isolating structures of interest by laser dissection prior to analyzing the protein compositions is useful, where not average protein compositions or concentrations are needed, but where specific biological structures need to be analyzed.

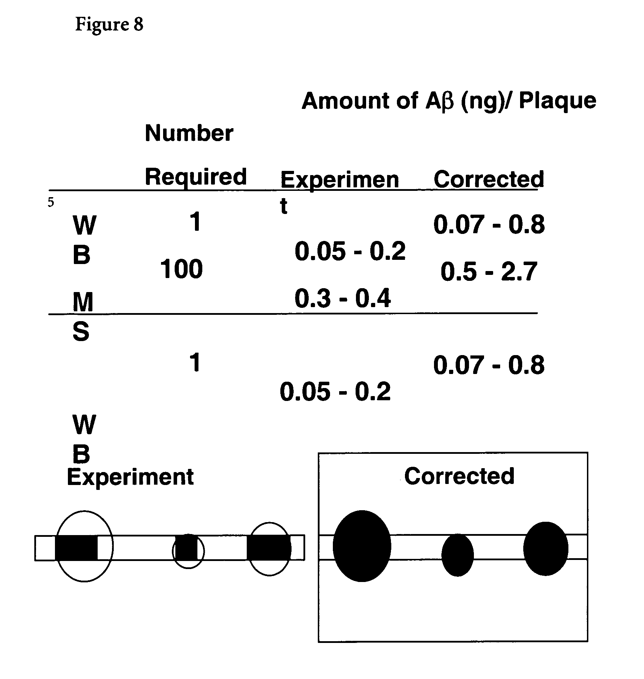

The excised amyloid deposits or plaques only represent a fraction of the whole, spherical plaque (Fig. 8). Therefore, the amount of Aβ determined in an excised plaque has to be balanced by a correction factor in order to arrive at the determination of the amount of Aβ present in a whole, spherical plaque.

Furthermore, after excision, the plaques may be transferred to a vessel by an electrostatic effect.

The tissue samples or body fluid may be a mammalian tissue sample or body fluid. More preferred are human and mouse tissue samples and body fluids.

The beta amyloid may be present in the source of beta amyloid in aggregated or in soluble form. While Aβ in plaques is known to incorporate into amyloid fibrils, soluble nonfibrillar forms of Aβ do exist *in vivo.* Teller et al. (Teller, J. K.; et al., Nat Med 1996, 2, 93-95) detected soluble Aβ species in aqueous extracts of brains from Down's syndrome subjects and normal aged controls; the samples were obtained at autopsy from fetuses and from subjects ranging in age from 4 days to 61 years old. The amount of soluble Aβ was several-fold greater in the Down's syndrome subjects, and it increased with age. Furthermore, the elevation of soluble Aβ occurred well in advance of neuritic plaque formation. Kuo et al. (Kuo, Y. M.; et al., J Biol Chem 1996, 271, 4077-4081) examined aqueous extracts of brains from 8 AD subjects and 4 normal controls, and found a 6-fold increase in the amount of soluble Aβ. Ultrafiltration experiments on the soluble Aβ indicated the presence of Aβ oligomers.

The presence of beta amyloid in the tissue sample or body fluid may be determined by methods comprising protein biochemistry, histochemistry and immunochemistry. Preferably, the presence of the aggregated beta amyloid in a tissue sample is determined by histochemical methods comprising staining with Congo Red or Thioflavin S, or by immunohistochemical methods. More preferably, the presence of the aggregated beta amyloid in a tissue sample is determined by double staining with histochemical and immunohistochemical methods. Most preferably, the presence of the aggregated beta amyloid in a tissue sample is determined by staining first with Congo Red followed by immunohistochemistry. Preferably, the presence of beta amlyoid in body fluid is determined by Western blotting of a body fluid sample.

The beta amyloid peptide which is to be quantified by the methods of the present invention may be the more soluble form of Aβ, Aβ1-40, which is normally produced in larger amounts by the cells. Additionally, it may be the Aβ1-42 form, ending at amino acid 42, which in contrast is the more hydrophobic form of Aβ found in neuritic plaques. The Aβ1-40 is usually colocalized with Aβ1-42 in plaques. Further forms of beta amyloid to be quantified by the methods of the present invention comprise Aβ1-38, Aβ1-39, Aβ1-43, as well as the N-terminal truncated forms Aβ3-40, Aβ3-42, Aβ4-42, Aβ6-42, Aβ7-42, Aβ8-42, Aβ9-42, Aβ11-42 (J Näslund, A Schierhorn, U Hellman, L Lannfelt, AD Roses, LO Tjernberg, J Silberring, SE Gandy, B Winblad, PG gard, C Nordstedt, and L Terenius (1994), Relative Abundance of Alzheimer Aβ Amyloid Peptide Variants in Alzheimer Disease and Normal Aging, PNAS 91: 8378-8382). The terms beta amyloid and Aβ are used equivalently in the present invention. The aggregated amyloid beta may be amyloid fibrils folding into "beta-pleated" sheet fibrils where amyloid fibrils are classified by the following criteria comprising (1) demonstration of Congo red binding and the display of green birefringence when viewed between crossed polarizers; (2) electron microscopic demonstration of fine nonbranching fibers, 6-10 nm in diameter; (3) presence of characteristic-structure; and (4) an x-ray fiber diffraction pattern resembling that of the cross- pattern seen in silk fibroin.

In the methods of the present invention, Aβ labeled with a stable isotope is added as a standard to the source of Aβ before the start of the dissolution and/or isolation procedure. Aβ labeled with a stable isotope is added directly to the homogenized tissue sample, directly to the excised amyloid deposit or directly to the body fluid sample.

The advantage of the method of the present invention is the fact that the beta amyloid standard labeled with a stable isotope can be spiked at the very beginning into the source of beta amyloid, e.g., into excised amyloid deposits or into a sample of body fluid. As the unlabeled Aβ to be quantified and the labeled Aβ standard are chemically identical except for mass difference in identical atoms, they behave identically in the required dissolution and/or isolation procedure of the aggregated amyloid or of soluble amyloid which may be bound by other proteins, which results in equal losses of the analyte and the standard.

The Aβ labeled with a stable isotope and added as a standard represents the same Aβ form as the one which is to be quantified in the source of Aβ. Therefore, the Aβ labeled with a stable isotope may be selected from the group comprising Aβ1-38, Aβ1-39, Aβ1-40, Aβ1-42, Aβ1-43, Aβ3-40, Aβ3-42, Aβ4-42, Aβ6-42, Aβ7-42, Aβ8-42, Aβ9-42, and Aβ11-42. The Aβ standard is labeled with at least one stable isotope selected from the group comprising ²H, ¹³C, ¹⁵N, and ¹⁸O. Preferably, the Aβ standard is labeled with ¹⁵N or ¹³C. More preferably, the Aβ standard is labeled with ¹⁵N. Preferably, the Aβ standard is labeled with as many stable isotopes as necessary for the separation of the isotope patterns in the mass spectra.

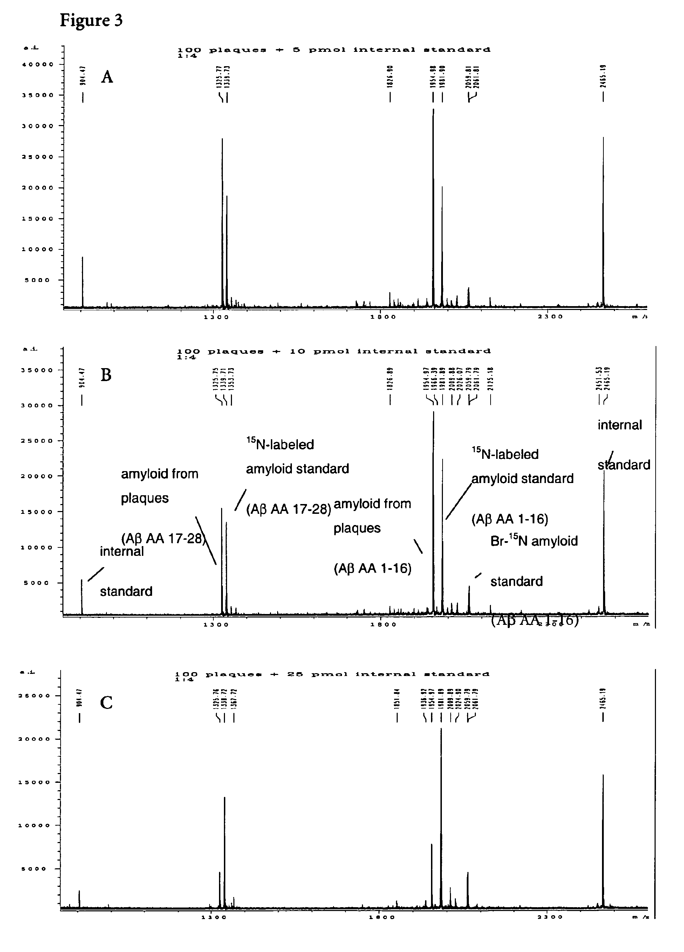

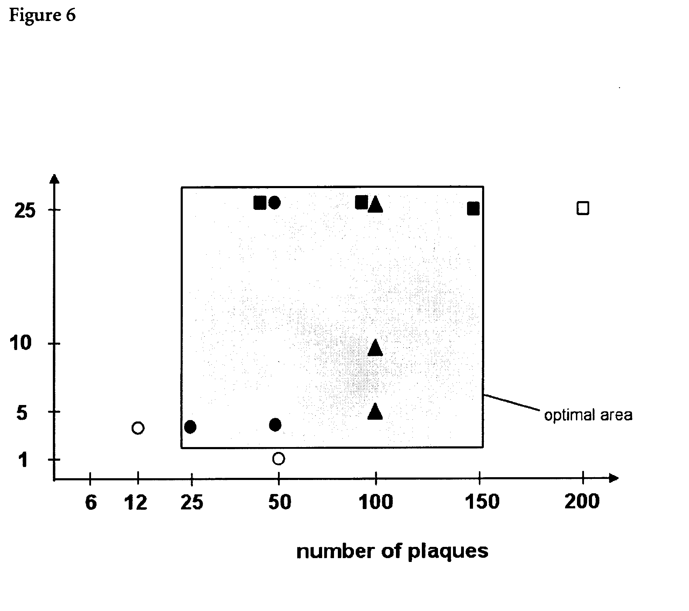

The Aβ standard labeled with a stable isotope is added in a defined amount. Preferably, the labeled Aβ standard is added in an amount in the same range as the effective amount of Aβ present in the source of beta amyloid. This amount may be determined in preliminary experiments, e.g. to find this amount for the quantification of Aβ in amyloid deposits, different numbers of plaques spiked with different amounts of Aβ standard have been analyzed (Fig. 6) in three successive experiments. Using 15 plaques or below, only the spiked ¹⁵N-labeled Aβ standard could be detected while results obtained from 200 plaques were found to exceed the instrumental linear range. In a third experiment, hundred plaques were spiked with 5, 10, and 25 pmoles of ¹⁵N-amyloid standard (Fig. 3). Using these conditions, a linear progression of the ¹⁴N/¹⁵N amyloid ratio could be observed: 10 pmoles of ¹⁵N-labeled Aβ standard nearly equaled the effective amount of Aβ present in 100 plaques (Fig. 3B) whereas the response obtained from 5 pmoles (Fig. 3A)and 25 pmoles ¹⁵N Aβ (Fig. 3C)was below, respectively above this amount. More preferably, the labeled Aβ standard is added in an amount which allows measurement in the linear measurement range of the mass spectrometer.

The Aβ labeled with a stable isotope used as a standard in the method of the present invention may be produced recombinantly. Methods for the preparation of expression constructs and for the recombinant production of polypeptides and proteins are known in the art and are summarized in Ausubel, Current Protocols in Molecular Biology / Protein science, Green Publishing Associates and Wiley Interscience, N.Y.(1994). Methods for the recombinant production of natural Aβ are described in the art, e.g. in EP0641861. Preferably, the labeled Aβ may be produced by feeding recombinant *E. coli* with ¹⁵N ammonium chloride. Other sources for stable isotopes comprise ¹³C-labeled glucose and extracts of algae grown on ¹⁵N-labeled substrates.

The Aβ labeled with a stable isotope used as a standard in the method of the present invention may be produced by chemical synthesis. Methods for the synthetic production of polypeptides and proteins are known in the art, e.g. solid phase synthesis of polypeptides, and are summarized in Ausubel, Current Protocols in Protein science, Green Publishing Associates and Wiley Interscience, N.Y.(1994). The demonstration that amyloid fibrils formed *in vitro* using synthetic Aβ peptides are identical to those isolated from senile plaques (Kirschner, D. A.; Inouye, H.; Duffy, L. K.; Sinclair, A.; Lind, M.; Selkoe, D. J. Proc Natl Acad Sci USA 1987, 84, 6953-6957) has validated the use of synthetic peptides in different studies. Solid phase peptide synthesis is the most common method used to prepare the synthetic peptides, and successful syntheses have been obtained using both Fmoc (9-fluorenylmethyloxycarbonyl) (Burdick, D; et al., J Biol Chem 1992, 267, 546-554) and Boc (t-butyloxycarbonyl)( Barrow, C. J.; et al., J Mol Biol 1992, 225, 1075-1093) methods for alpha-amino protection. While the Aβ peptides are moderately difficult to synthesize, standard coupling methods and side-chain protection strategies have proven to be sufficient for successful synthesis. For the introduction of stable isotopes into the Aβ standard, amino acids labeled with a stable isotope are used in the synthesis methods.

After addition of the Aβ standard, total Aβ comprising labeled and unlabeled Aβ may be isolated from body fluid, preferably from serum or CSF, by protein chemical methods comprising immunoprecipitation and immunoaffinity chromatography.

Therefore, in a further embodiment, the beta amyloid in step (c) is isolated from body fluid by methods comprising protein chemistry and immunochemistry.

For the determination of the Aβ content of a source of Aβ containing aggregated Aβ, the aggregated Aβ has to be dissolved. In the method of the present invention, the aggregated Aβ is dissolved by methods comprising dissolution with solubilizing agents, and optionally mechanical solubilisation in the presence of the labeled Aβ standard. The solubilizing agents of the present invention may be all agents which have the capacity to dissolve aggregated Aβ, e.g. hexafluoropropanol, acid, e.g. formic acid, urea-SDS. The mechanical solubilisation may comprise sonication. The dissolution procedure of the aggregated Aβ takes place in the presence of the added labeled Aβ standard thereby guaranteeing equal losses of the Aβ standard and the Aβ to be quantified.

Therefore, in a further embodiment, the beta amyloid in step (c) is isolated from amyloid deposits by methods comprising dissolution with solubilizing agents and optionally by sonication.

The isolated Aβ is subsequently prepared for analysis by mass spectrometry. The preparation for the analysis by mass spectrometry comprises methods which lead to an amelioration of the ionisation of the Aβ to be analysed. Methods leading to an amelioration of ionisation comprise fragmentation by methods comprising chemical fragmentation and enzymatic digestion, and chemical reactions with flight enhancers. Chemical reactions with flight enhancers comprise the charge derivatization of the peptides' free N-termini in order to enhance sensitivity and promote the formation of fragment ions by post-source decay MALDI mass spectrometry (J. Stults et al. (1993) Anal. Chem. 65, 1703-1708; B. Spengler et al. (1997) Int. J. Mass Spectrom. 169-170, 127-140; Z. Huang et al. (1999) Anal. Biochem. 268, 305-317; Staudenmann W. and James P. in Proteome Research: Mass Spectrometry (P. James, Ed) Springer Verlag, Berlin (2001) 143-166). The isolated Aβ may be directly reacted with flight enhancers for preparation for analysis by mass spectrometry. Alternatively, the isolated Aβ may be reacted with flight enhancers after fragmentation. Chemical fragmentation may be carried out by the use of cyanogen bromide or acid hydrolysis. The enzymatic digestion may be carried out with a protease selected from the group comprising endoproteinase Lys-C, trypsin, endoproteinase Glu-C, and pepsin. In the method of the present invention, the isolated Aβ may be dried and redissolved in a buffer before digestion with a protease. The fragmentation of the dissolved Aβ leads to a better limit of detection in the mass spectrometrical analysis, e.g., cleavage of the amyloid peptide by endoproteinase Lys-C results in the detection of two of the three generated fragments with a 100 fold higher sensitivity in the mass spectrometer.

Therefore, in a further embodiment, the isolated beta amyloid in step (d) is prepared for analysis by mass spectrometry by methods comprising chemical reactions with flight enhancers, chemical fragmentation and enzymatic digestion.

Before mass spectrometrical analysis, the dissolved and optionally fragmented Aβ may be desalted. Desalting of the sample (e.g., by ZipTip) may increase the sensitivity of the mass spectrometrical analysis by a factor of 10.

The isolated and optionally fragmented Aβ is then analysed by mass spectrometry. Ionization techniques used for biological materials today comprise ESI (electrosprayionization) and MALDI (matrix assisted laser desorption ionization). Preferably, the mass spectrometrical analysis used is a MALDI-TOF (time of flight) mass spectrometrical analysis. The spectrum of a MALDI-TOF-MS analysis consists primarily of the intact, singly charged molecule ions. Larger molecules, like proteins, may also yield multiply charged ions and, depending on their concentrations, singly charged multimers.

The peak pattern of the natural ¹⁴N Aβ is base-line separated from its artificial ¹⁵N homologue in mass-spectrometry, thereby allowing the discrimination of the natural and the standard Aβ in the mass spectra.

The amount of Aβ that was present in the source of aggregated Aβ may be determined by different approaches to the analysis of the mass spectra: a) by comparing the heights of the two dominant peaks of the ¹⁵N-labeled amyloid standard and of the Aβ from the source of Aβ, b) by comparing the heights of all the peaks of the separated peak patterns, c) by comparing the areas under the two dominant peaks, and d) by comparing the sum of the areas under all the peaks of the two different peak patterns. With the defined and known amount of labeled Aβ standard added at the beginning of the procedure, the amount of Aβ present in the source of Aβ can then be calculated. In cases where the amount of Aβ that is present in a three-dimensional amyloid deposit, e.g. in a plaque, has to be determined a correction factor has to be included in the calculation.

In a further embodiment of the present invention, a method for the quantification of beta amyloid peptide is provided comprising
(a) providing excised amyloid deposits from mammalian brain samples containing aggregated beta amyloid
(b) adding a defined amount of beta amyloid peptide labeled with a stable isotope
(c) dissolving the excised aggregated beta amyloid in the presence of the labeled beta amyloid
(d) digesting the dissolved beta amyloid with a protease
(e) analysing the digested beta amyloid peptide mixture by mass spectrometry, and
(f) determining the amount of beta amyloid that was present in the source of aggregated beta amyloid with the help of the base-line separation resulting from the presence of the natural and the stable isotopes in the beta amyloid.

### Applications

The present invention further provides the use of the method of the present invention for the determination of the Aβ content in amyloid deposits, e.g., plaques, obtained from tissue samples. A method of quantifying amyloid deposition before death is needed both as a diagnostic tool in mild or clinically confusing cases as well as in monitoring the effectiveness of therapies targeted at preventing Aβ deposition.

Additionally, the use of the method of the present invention for the determination of the Aβ content in body fluid containing soluble Aβ, e.g. serum or CSF, is provided. Different observations have led to an expansion of the amyloid hypothesis, which includes soluble forms of Aβ among the neurotoxic species responsible for the pathology of AD.

Furthermore, the method can be used for the quantification of amino terminal and carboxy terminal beta amyloid microheterogeneities. This is accomplished by spiking the source of beta amyloid with the specific form of beta amyloid the amount of which should be determined.

In case of cerebral amyloidosis the distribution of different forms of Aβ (e.g. 1-40, 1-42, 1-39, 1-43) in the brain could be clarified. Using laser dissection microscopy, different structures of AD brains (blood vessels, dense plaques, diffuse plaques) can be selectively excised and analyzed with mass spectroscopy. In addition, individual protein compositions can be analyzed in these different structures.

Having now generally described this invention, the same will become better understood by reference to the specific examples, which are included herein for purpose of illustration only and are not intended to be limiting unless otherwise specified, in connection with the following figures.

### Examples

Commercially available reagents referred to in the examples were used according to manufacturer's instructions unless otherwise indicated.

### Identification of amyloid plaques by histochemistry

### Transgenic animals and human brain specimen

The employed double transgenic mice have an APPS^{SWe}/PS2 background and were 20 months old (Richards J.G., Messer J., Goepfert F., Ozmen L., Brockhaus M., Bohrmann B., Malherbe P., Jacobsen H., Huber G.S., Bluethmann H., Kew J.N.C., Kemp J.A. Ouagazzal A.M. and Higgins G.A. (2001), Double transgenic mice overexpressing hAPP^{SWe} and hPS2mut show age-dependent cognitive deficits and amyloid deposits in discrete brain regions, Soc. Neurosci. Abstr., Vol. 27, Program No. 546.7, 2001.).

The human brain tissue employed in the performed experiments was obtained from a single donor after informed consent of patient was obtained. The post mortem tissue was collected from a single Alzheimer's patient only two hours after death and instantly frozen (-80°C) to conserve the native tissue structures. The patient had a ApoE4/E4 genetic background and the sample was taken from cortical areas.

### Preparation of slices from mouse and human brain samples

Mice were killed either by cervical dislocation or by decapitation after anesthesia with halothane. The skullpan was opened with scissors, the brain was removed and divided into hemispheres before freezing in dry ice. All animal experiments were performed in full accordance with the guidelines issued by the responsible Swiss Veterinary Office.

For (immuno-) histochemistry, the brain tissue was cut into slices with a thickness of 10 µm with a kryostat microtome (LEICA CM3050 S). These slices were placed on a glass coverslip and were stored for further processing (e.g. staining) at -20°C.

For slices to be submitted to laser dissection microscopy, special coverslips coated with a 1.35µm thick polyethylene foil (P.A.L.M., LPC-MOMenT-Object slides, 8150) were used.

### Congo Red staining

Staining with Congo Red was then performed using a commercially available staining kit (Sigma Diagnostics, Accustain Amyloid Stain, Congo Red, HT60).

Brain tissue sections were rehydrated for 5 minutes in PBS. Cell nuclei were stained with Mayers Hematoxylin solution (Fluka, Hematoxylin Mayer-solution, 51275) by incubation in a glass cuvette for 10 minutes at room temperature. This solution stains the nuclei during 10 minutes. After washing and rinsing the probes with tap water for 5 minutes, they were treated with alkaline sodium chloride solution for 20 minutes. This solution has to be prepared first by adding NaOH (1/100 of the volume of NaCl; Sigma Diagnostics, sodium hydroxide solution, HT60-2) to the sodium chloride (Sigma Diagnostics, sodium chloride solution, HT60-1) solution. Following that, the probes were treated for 20 min with.alkaline Congo Red solution (Sigma Diagnostics, Congo Red solution, HT60-3), which has to be prepared as well in advance by adding 1% NaOH and by filtering the solution (thereby removing crystals). The following washings (2x) with ethanol (Merck, Ethanol, 100983) removed the unbound Congo Red. Rinsing the slices with Xylol (Fluka, m-Xylol, 95673) was the last step, before embedding the samples with a fluorescent mounting medium (DAKO, Fluorescent Mounting Medium, S3023).

Congo Red, like Thioflavine S, is a stain for beta-pleated sheet secondary structures of proteins, like those present in amyloid fibrils and can be used to visualize dense plaques that are characteristic for Alzheimer's Disease (Carter et al (1997), A Model for Structure-Dependent Binding of Congo Red to Alzheimer β-Amyloid Fibrils, Neurobiology of aging 19: 37-40).

The Congo Red (CR) staining revealed in humans as in mice dense packed plaques, consisting of the Aβ peptide. However, human and transgenic mouse brain sections differed in several aspects when staining them with CR. Stained brain sections of 20 months old mice revealed a generally higher number of CR-reactive spots compared to an area of the same size in the neocortex of the human brain sample. The stained amyloid plaques in mice were distributed homogeneously over cortical areas, whereas in the used human case only few areas with few congophilic plaques were found. Human plaques were slightly bigger than mouse plaques from the employed transgenic model.

From a multitude of brain sections and images it emerged that the majority of mouse plaques is stained more intensely and appear more compact than human dense plaques.

### Thioflavine S staining

Brain tissue slices were rehydrated for 5 minutes in phosphate buffered saline (PBS). After removal of the PBS, an aqueous Thioflavine S (Sigma, Thioflavine S, T1892) solution (1%) was applied for 3-5 minutes. After that, the probes were allowed to differentiate for 3-5 min in 70% ethanol (Merck, Ethanol, 100983). The last, mounting was done with glycerol-H₂O (3:1) (FLUKA, Glycerol anhydrous, 49770).

Since the sensitivity of Thioflavine S staining is comparable to that of Congo Red, the observations concerning plaque size, plaque number and intensity of the spots could be confirmed under the fluorescent microscope. An additional difference between human and the transgenic model was the staining of vessels. Staining human brain samples with Thioflavine S (or CR) revealed occasionally a strong fluorescent signal in brain blood vessels. When staining mouse brain samples from the employed double transgenic model with Thioflavine S, no such deposits could be observed.

### Immunostaining for Aβ

Brain tissue slices were rehydrated for 5 minutes in PBS. After the removal of the PBS, 1ml 70% acetone at 4°C (Fluka, Acetone, 00570) was applied to the brain tissue slices for approximately 80 seconds. The sections then were washed twice for 2 minutes with 1ml PBS and then, non specific binding sites were blocked for 15 minutes with 500µl PBS containing 1 % BSA (Roche, Bovine Serum Albumine Fraction 5, 775869), 1 % Ovalbumine (Fluka, Albumine from hen egg white, 05440) and 1% Normal Goat Serum (BBInternational, Normal Goat Serum, NGS5). After finishing the blocking step, the samples were treated with 200µl of the primary antibody (F. Hoffmann-La Roche Ldt., BAP-2, ID 358, recognizes AA 2-8 of Aβ), diluted 1:10 in blocking solution to a final concentration of approximately 3µg/ml, for 30 minutes at room temperature. Washing with 500µlPBS + 1% BSA (3 x 5 minutes) followed this incubation. For detection, a secondary antibody (Molecular Probes, Alexa Fluor 488, Goat Anti-Mouse, A-11001, ID: 555), diluted 1:200 in PBS +1% BSA, was then applied for 30 minutes at room temperature. Two washing steps for 5 minutes with 1ml PBS and one washing step for 2min with H₂O-Bides prepared the probe for the mounting step with an embedding medium (DAKO, Fluorescent Mounting Medium, S3023). The samples were then examined under the fluorescence microscope and stored at 4°C.

If the samples were to be used for laser dissection, the PBS employed during immunostainig had to contain protease inhibitor (1 Tab/ 50ml, Roche Diagnostics, Protease inhibitor cocktail tablets, 1836145). The slides were air-dryed and stored until further usage at -20°C.

The most sensitive method to stain Aβ depositions is the labeling with specific monoclonal or polyclonal antibodies against the peptide. Staining characteristics of brain sections were also different according to the used antibody. Human and transgenic mouse brain samples did again not reveal the same appearance when stained with antibodies. Human and mouse plaques were, in addition to the previously described difference in size and number, different with regard to diffuse depositions of Aβ that are exclusively detected by immunohistochemistry.

Brains from transgenic mice appear to be overloaded with the human Aβ peptide and hence, large brain regions of the employed transgenic mice were covered with large, diffuse patches of various sizes that were not found in human brain samples. The certainty that these depositions really were amyloid depositions, was obtained by analyzing such regions by Western blotting. The fact that large brain regions nearby this diffuse immunoreactivity were totally free from any fluorescent signal further confirmed this observation.

Additionally, dense and diffuse plaques in mice had a different morphology and could be distinguished clearly, whereas this distinction is not as clear in humans most likely due to continuous transition between dense and diffuse plaques.

### Double Labeling

In principle, the process for double labeling is the combination of Congo Red staining (or Thioflavine S staining) and the immunolabeling. After testing the two possible sequences of the staining methods, best results were obtained by Congo Red staining first, then followed by immunostaining. The staining procedures were sequentially performed as described above by performing the acetone treatment right after the rehydration with PBS.

To test the presence of plaques in the available biological material, brain samples from transgenic mice and human brain were stained with Congo Red, Thioflavine S and specific antibodies against Aβ.

The staining properties of Aβ from human and transgenic mice are summarized in Table 1.

The observed differences in the (immuno) histochemistry of amyloid depositions was investigated by a doublestaining approach. This procedure revealed prominent Aβ depositions, which were not marked by Congo Red/Thioflavine S but recognized by the antibody. To show that the smaller number of congophilic spots in humans is not due to the absence of Aβ depositions, but is indicative to a difference of sensitivity of Congo Red/Thioflavine S and the more sensitive antibody. The observed difference in stainability with tinctorial stains compared to immunohistochemistry is likely due to differences in compactness of human and transgenic mouse plaques.

These stainings showed, that at least two different kinds of Aβ depositions occur frequently in human (and transgenic mouse) Alzheimer's disease brains, namely dense and diffuse plaques. The less sensitive Congo Red only stained the very compact plaques, whereas the more sensitive antibody in addition revealed diffuse depositions.

**Table 1:**

| Comparison of histopathological observations in human and transgenic mouse brain samples | | | |
|---|---|---|---|
| | Human Brain Sample | Transgenic Mice | Conclusions |
| **Congo Red Staining (CR)** | - occasionally areas with few CR-reactive spots - small number of CR-positive plaques - staining of vessels (microangiopathy) | - CR-positive plaques homogenously distributed over cortical areas - large number of CR-positive plaques; many are small - few are big like in the human case - there is size variation | - human plaques are bigger than transgenic mouse plaques - transgenic mouse brain sections contain more CR-positive plaques - plaques in humans seem to be less dense packed with Aβ fibrils - microangiopathy found in humans but not in the employed transgenic mouse model |
| **Thioflavine S Staining** | - number and size of marked spots comparable to CR-staining | - number and size of marked spots comparable to CR-staining | - conclusions as CR staining |
| **antibody / double labeling** | - large number of Aβ-depositions - diffuse depositions - double labeling shows two types of depositions: Dense and diffuse plaques with intermediate stages | - brain is overloaded with amyloid depositions - heterogeneous patches of diffuse Aβ depositions - clear difference between dense and diffuse depositions | - in humans, the transition from diffuse to dense plaques is a continuum, whereas in mice, basically two different types can be distinguished - small number of CR-positive spots in humans together with frequent diffuse Aβ depositions |

### Harvesting and processing of amyloid plaques

### Laser dissection microscopy

The investigation of plaque proteins depended on a sophisticated procedure for isolation of plaques and sensitive protein chemistry methods. For isolation, the laser dissection microscopy (LDM) was applied (Schütze et al (1998), Identification of expressed genes by laser-mediated manipulation of single cells, Nature Biotechnology 16: 737-742; Simone et al (1998), Laser-capture microdissection: opening the microscopic frontier to molecular analysis, TIG 14: 272-276). The employed laser dissection microscope (P.A.L.M., Robot-MicroBeam) consists of three main components:

An inverse fluorescence microscope (Carl Zeiss, Axiovert 135) with newly developed filters (beam splitters), which apart from selecting the appropriate excitation wavelength, let also pass the laser beam and filter the desired emission wavelength (Zeiss, Filter set 09, exitation: BP 450-490, beamsplitter: FT 510, emission: LP 520)

A pulsed nitrogen laser (337nm), which can be varied with respect to its focus (centering the laser beam into the focal plane of the objective) and with respect to the energy of the laser. The laser is adjusted for cold ablation using a maximum setting of 30 energy pulses per minute with a pulse duration of 3ns. Compared to a steady laser beam, a pulsed laser avoids the convection of excessive heat that might damage the biological specimen

A computerized system, which allows the control of a motorized xy-stage for automated positioning of the laser beam and the storage of up to 50 dissection positions. The sample can be observed and investigated through the microscope or, alternatively using a CCD camera, on a monitor.

The laser dissection microscopy comprises the steps of cold ablation and laser pressure catapulting necessitating two different settings of the laser beam:

### 1) Cold ablation

The excision process is a restricted ablative photodecomposition process without heating (Srinivasan R., (1986) Ablation of polymers and biological tissue by ultraviolett lasers, Science 234: 559 - 565). The brain slices first were dried at 37°C for ~30 minutes. For actually excising a biological structure, the microscope was set at a 20-fold magnification and the focus was set up at ~35 points and the energy at ~44 points (arbitrary units). After excision of the desired structure, the position is stored by the simple push of a button.

### 2) Laser Pressure Catapulting (LPC)

In the second step, the excised samples were collected. This step is technically mediated by a single laser pulse of increased energy (+20 points), which has its focal plane slightly below the sample (-2.2 points, ~ 1-2µm). A single laser pulse provokes a rapid gas expansion that transports the excised material directly into the cap of a common microfuge tube, which is held and centered above the line of laser fire by a special LPC-collector device. By this procedure, the excised material was accumulated in the cap.

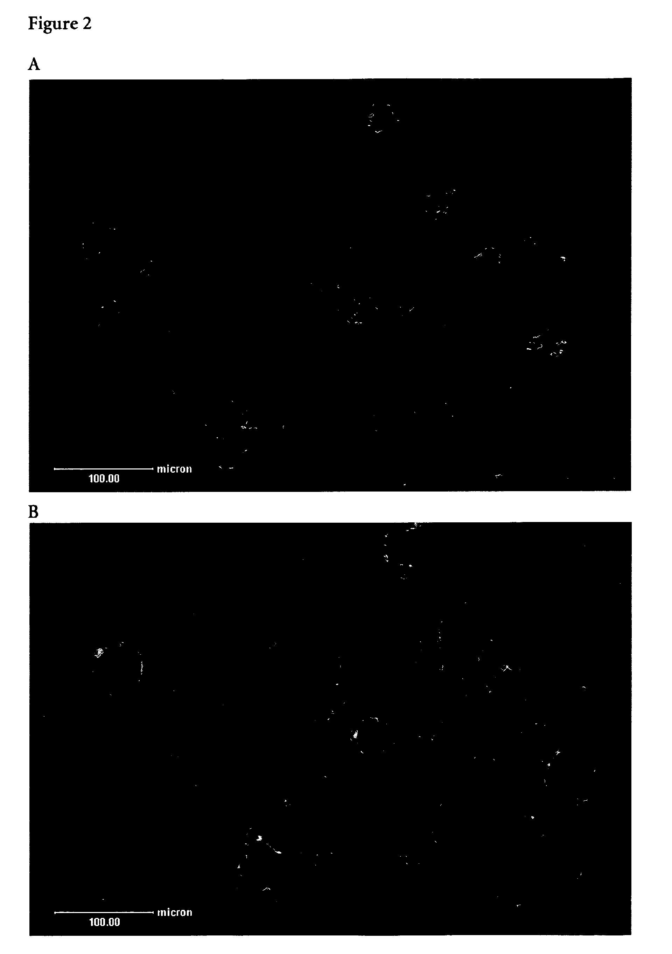

The excized material cut out from a brain section does not represent the entire plaque, but only a disk out of the three-dimensionally shaped amyloid deposition (Fig. 8). This fact has then to be taken into account when determining the amount of Aβ present in a three-dimensionally shaped amyloid deposit. Pictures of an immunostained transgenic mouse brain section before and after excision of plaques by LDM are shown in Fig. 2A and Fig. 2B, respectively.

The cap was filled with ~25µl sample buffer (for mouse tissue: Invitrogen, NuPAGE SDS Sample Buffer 4x, NP0003) containing 8M urea (BioRad, Urea, 161-0731) or ~25µl formic acid (for human tissue: Fluka, formic acid, 06440). Following collection of excised tissue, the liquid was spun down at 15'500 x g. The cap with the human samples was additionally washed out two times with 30µl HCOOH and spun down. The human material was left in HCOOH overnight. Then it was vortexed, sonified 3 x for 3min in a bath with vortexing in between. After that, the HCOOH (~90µl) was evaporated in an evacuated centrifuge to dryness. To neutralize the acidic residue, 50µl of a 1% pyridine solution was added, vortexed and evaporated again in an evacuated centrifuge to dryness. The remaining residue was combined with 25µl SDS sample buffer containing 8M urea and was treated further as described in the Western blot assay below.

### Analysis and Quantification of beta amyloid

### Detection of Aβ by mass spectrometry

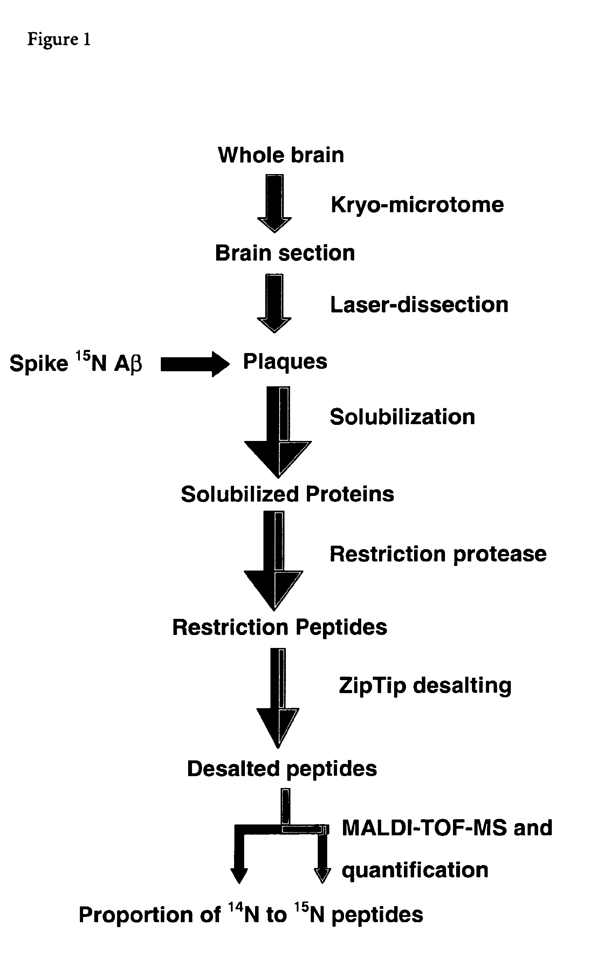

One hundred plaques from a stained mouse brain section were excised by laser dissection and treated with formic acid overnight. The following day, the samples were sonicated for 3x3 min with vortexing in between. After spinning down the liquid at 11900 x g for 5min, the samples were evaporated to dryness in a Speed Vac. The obtained pellet was digested in 10µl of 10mM ammonium bicarbonate containing 0.1-1µg lys-C or trypsin at room temperature overnight. The samples were desalted using ZipTip^{TM} (MILLIPORE, ZipTip, ZTC18S960) before being spotted onto the MALDI target. The procedure is summarized in Fig. 1.

The following measures had to be taken in order to avoid a background of keratin:
The kryostate was cleaned and plastic gloves were worn throughout the preparation of the brain samples to avoid contamination. The addition of goat serum to block unspecific binding sites in the brain section was omitted in the staining procedure. Additionally, the purified Aβ antibody rather than the BAP-2 ascites, (which was withdrawn with a syringe through the skin) was employed to stain Aβ-positive plaques.

### Quantification of Aβ by isotope dilution and mass spectrometry

The digestion pattern of the Aβ peptides obtained from amyloid plaques was compared to the proteolytic fragments obtained from ¹⁵N-labeled biosynthetic Aβ 1-42. ¹⁵N-labeled biosynthetic Aβ 1-42 was produced according to Doebeli et al. (Doebeli et al., Biotechnology 13, 1995, 988 - 993) using ¹⁵N-labeled amino acids. The Aβ standard was analyzed using the same work-up procedure as for plaque-derived Aβ.

To quantify the amount of amyloid in mouse plaques, a defined amount of ¹⁵N-Aβ1-42(M35Mox) was added to the lid of the Eppendorf tube containing the excised plaques. The resulting mixture was then analyzed by MALDI mass spectrometry using a Bruker Ultraflex Tof-Tof mass spectrometer (Bruker Daltonics, Bremen, Germany) operated in reflector mode using standard parameters.

Relative quantification by mass spectrometry is most accurate when the amount of the spiked protein (e.g. Aβ) standard equals the effective amount of protein present in plaques. To find this amount, different numbers of plaques spiked with different amounts of Aβ-standards have been analyzed in three successive experiments (Fig. 6). Using 15 plaques or below, only the spiked ¹⁵N-labeled Aβ-standard could be detected while results obtained from 200 plaques were found to exceed the instrumental linear range. In a third experiment, hundred plaques were spiked with 5, 10, and 25 pmoles of ¹⁵N-amyloid standard (Fig. 3). Using these conditions, a linear progression of the ¹⁴N/¹⁵N amyloid ratio could be observed: 10 pmoles of ¹⁵N-labeled Aβ-standard nearly equaled the effective amount of Aβ present in 100 plaques (Fig. 3B) whereas the response obtained from 5 pmoles (Fig. 3A) and 25 pmoles ¹⁵N Aβ was below (Fig. 3C), respectively above this amount.

By comparing the heights of two peaks of the ¹⁵N-labeled amyloid standard and of the amyloid out of plaques, the effective amount of (dissolvable) Aβ present in 100 plaques can be calculated and consequently the amount of Aβ in a single excised plaque can be determined.

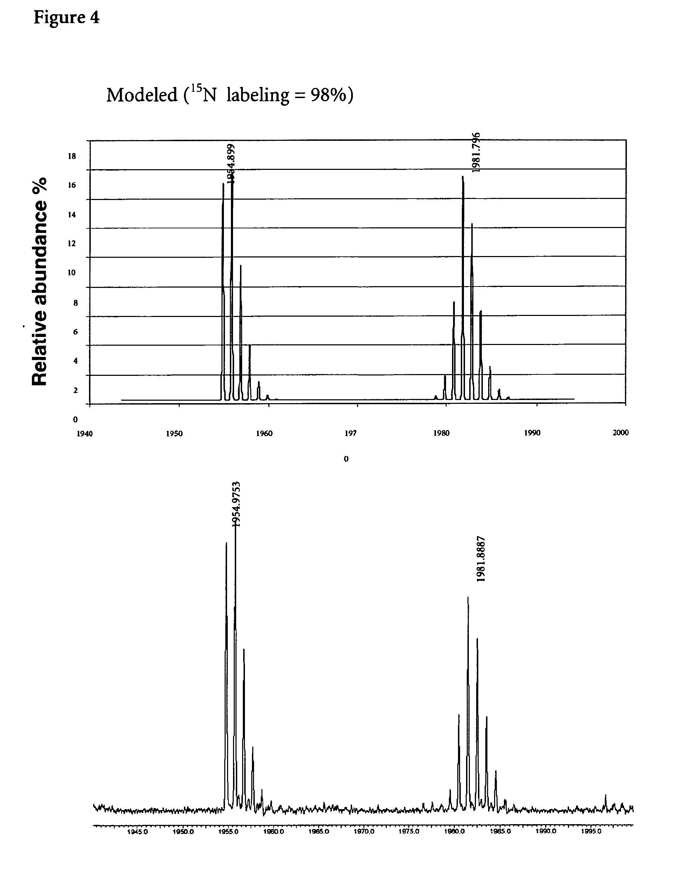

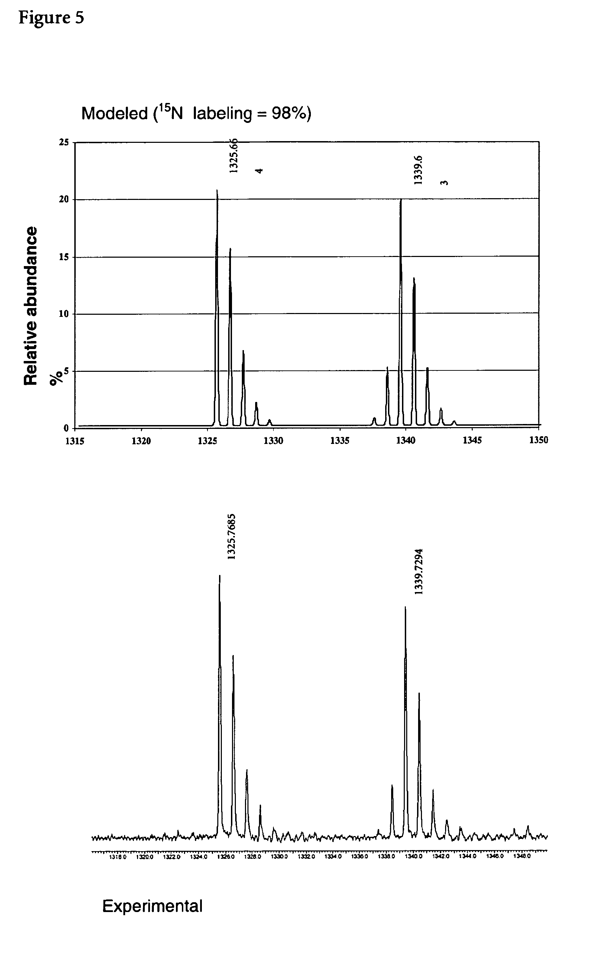

Comparison of the modeled isotopic distributions of the two Aβ fragments revealed, that the monoisotopic peaks (¹²C/¹⁴N and ¹²C/¹⁵N) had approximately similar heights (Fig. 4 and 5). Therefore, by comparing the heights of the analogous peaks obtained from the experiment, the amount of Aβ in plaques could be determined when the spiked amount of Aβ standard is known. These results are shown in Figures 8A.

### Qualitative confirmation of Aβ

### Tandem mass spectrometry

Confirmation that the detected peptides were originating from the plaque Aβ protein was achieved by tandem mass spectrometric analysis. The Aβ peptides obtained from the plaques after Lys-C digestion were isolated by time gating and fragmented by post-source decay. As peptides preferentially fragment at peptide bonds, the obtained fragmentation patterns are therefore representative of their amino acid sequences. The corresponding peptides obtained from the Lys-C digestion of a ¹⁴N-amyloid synthetic standard were similarly analyzed. Comparison of the tandem mass spectra obtained from the plaques' Aβ peptides with the tandem mass spectra from the Aβ standard revealed identical patterns, thereby confirming the presence of the Aβ protein in plaques.

### Isotopic distribution

An additional qualitative proof of the Aβ fragments was achieved by a comparison of the isotopic distribution. Each peptide (i.e. an Aβ fragment) has a characteristic distribution of naturally occurring isotopes (i.e. 12C / 13C, 14N / 15N). The mass spectrometric analysis of a fragment therefore reveals a characteristic set of peaks which, by comparison to the theoretically calculated isotopic distribution, clearly identifies a certain peptide.

Comparison of these modulated and experimentally obtained spectra of the isotopic distribution of the Aβ fragments 1-16 (Fig. 4) and 17-28 (Fig. 5), revealed identical patterns, thereby confirming qualitatively the occurrence of the Aβ protein in plaques.

### Scaling Aβ content in isolated plaque segments to entire plaques

The results presented here on estimated amounts of Aβ in plaques were extrapolated to the absolute content in entire plaques. As defined, the term 'plaque' was used for a slice of 10µm that has been cut out from the entire plaque with a diameter of ~40µm (mouse) up to ~70µm (human). To scale the amount of Aβ obtained for this disc to a whole, spherical plaque, a 'correction' factor that depends on the thickness of the cut and the size of the plaque, has to be calculated.

The ratio of volume of the disc to the total volume of the sphere of a plaque (V_{disc}: r² π h and Vₛₚₕₑᵣₑ: 4/3πr³) is the 'correction' factor to extrapolate the amount of Aβ determined in a disc to the entire amount in the sphere. In Table 2, the 'correction' factors for different plaque sizes, assuming a slice-thickness of 10µm, are listed:

**Table 2:**

| Correction factors for different plaques sizes | |
|---|---|
| radius of plaque | 'correction' factor (Vₛₚₕₑᵣₑ / V_{disc}) |
| 10µm | 1.34 |
| 30µm | 4 |
| 50µm | 6.67 |

### Validation of the method with Western blotting

Western blotting was performed according to Ida et al. (Ida N., Hartmann T., Pantel J., Schröder J., Zerfass R., Förstl H., Sandbrink R., Masters C. L., Beyreuther K. (1996) Analysis of Heterogenous βA4 Peptides in Human Cerebrospinal Fluid and Blood by a Newly Developed Sensitive Western Blot Assay, J Biol Chem 271: 22908-22914) with minor modifications.

The samples, containing the biological material in sample buffer (Invitrogen, NuPAGE SDS Sample Buffer 4x, NP0003) containing 8M urea (BioRad, urea, 161-0731), were mixed with 2µl of reducing agent (Invitrogen, NuPAGE Sample reducing agent 10x, NP0004), vortexed, heated at 50°C for 10min, again vortexed and heated to 50°C for 10min and centrifuged at 11900 x g for 5min.

Separation was done with by 10% SDS-PAGE (Invitrogen, 10% NuPAGE Bis-Tris-Gel, NP0301) with MES running buffer (Invitrogen, NuPAGE MES SDS Running Buffer, NP0002). Separated proteins on the gels were electrophoretically transferred (transfer buffer: Invitrogen, NuPAGE Transfer Buffer 20x, NP0006 + 20%MeOH (Merck, 106009); Transfer Blots: BioRad, Mini Trans-Blot Filter Papers, 170-3932) onto nitrocellulose membrane (Amersham, Hybond-C extra, RPN303E) at 25V for 1h. The blotted membrane was heated for 3min with microwaves (900W) in boiling PBS to enhance the binding, and the unspecific binding sites were blocked with 50ml 5% skim milk (FLUKA, Skim Milk Powder, 70166) in PBS containing 0.05% Tween20 (Fluka, Tween20, 93773), PBS-T, for at least 30min. After rinsing (2x) and washing (1x5min) the membrane with fresh PBS-T, the WO-2 antibody (provided by the lab of K. Beyreuther, Center for Molecular Biology, University of Heidelberg; providing a strong signal without any background) diluted in PBS-T was added and incubated overnight at 4°C. The membrane was first washed with PBS-T, then soaked (3x10min) in fresh PBS-T and the bound antibody detected by 50ml of a horseradish peroxidase linked secondary antibody (Amersham, NA931/NA934) diluted 1:10'000 in PBS-T. The same washing and soaking cycle with PBS-T was applied to the membrane as before. Visualization was performed by ECL detection system (Amersham, ECL Western blotting detection reagents, RPN2106) according to the manufacturer's instruction by exposing the membrane to an autoradiography film (Kodak, BIOMAX ML, 243 012 06).

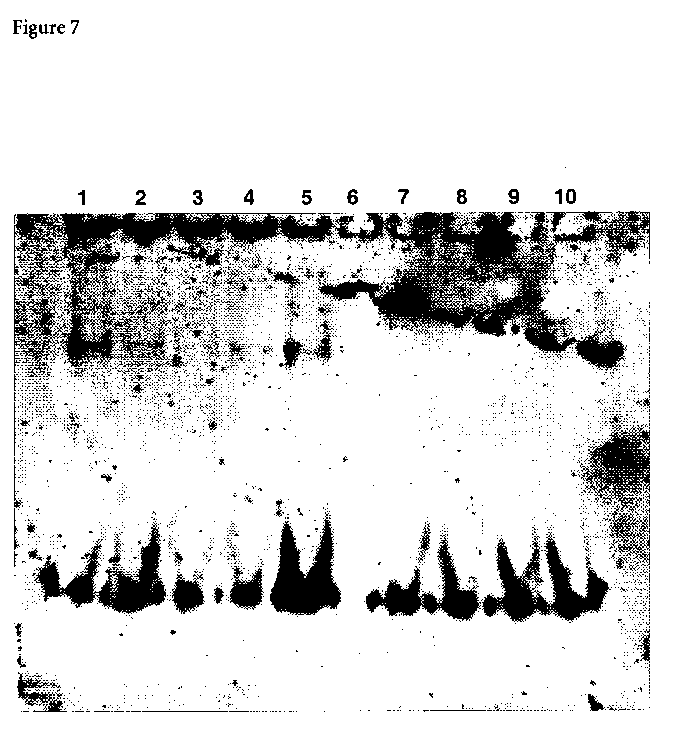

By loading different numbers of plaques on the gel, the minimum number of mouse plaques for Aβ detection by Western blot was determined to be one single plaque (Fig. 7). The intensities of the detected bands varied from plaque to plaque. To obtain an estimate of the Aβ content, several plaques were loaded on a gel and compared with band intensities of standard concentrations of synthetic Aβ. The majority of mouse plaques contained amounts of Aβ that were within a range of 0.05-0.2ng. According to the measured plaque sizes, the radius of a dense mouse plaque (spherical) is between 10 and 30µm. With an estimated amount of 0.05-0.2ng Aβ in an excised plaque (disc), a total amount of 0.07ng to 0.8ng Aβ in an entire mouse plaque (spherical) can be calculated.

By comparing the intensity of the bands from 0.1ng synthetic Aβ with the bands from the different number of human plaques, the amount of Aβ in a single human plaque was estimated. Whereas 9 human dense plaques contained more than 0.1ng Aβ, 4 human dense plaques were below this mark. Therefore, the amount of Aβ in a single human plaque is around 0.01ng. The radius of a human plaque is between 10 and 50µm, the amount of Aβ between 0.01 and 0.05ng. Thus, the total amount of Aβ in an entire human plaque (spherical) can be calculated to be in the range between 0.013 and 0.33ng.

By comparing the quantification results of beta amyloid present in mouse plaques obtained by MS and by Western blot, the method comprising MS and isotope dilution detected a ~ 4-5 fold higher amount of Aβ present in a single mouse plaque. This is due to the fact that a huge proportion of the analyte is lost during the procedure. In the mass-spectrometry method the loss of ¹⁴N and ¹⁵ N Aβ is assumed to occur at the same rate, and the proportion of these two isotope species reflects the situation at the beginning of the procedure. In Western blot (or ELISA) no internal standards can be used and the loss of analyte will be an unknown factor.

## Claims

1. A method for the quantification of beta amyloid peptide comprising
(a) providing a source of beta amyloid
(b) adding a defined amount of beta amyloid peptide labeled with a stable isotope to the source of (a)
(c) isolating unlabeled and labeled beta amyloid
(d) preparing the isolated beta amyloid for analysis by mass spectrometry
(e) analysing the prepared beta amyloid peptide by mass spectrometry, and
(f) determining the amount of beta amyloid that was present in the source of beta amyloid.

2. The method according to claim 1, wherein the source of beta amyloid in step (a) are amyloid deposits obtained from a tissue sample.

3. The method according to claim 2, wherein the amyloid deposits are obtained from a tissue sample by excision by laser dissection microscopy.

4. The method according to claim 1, wherein the source of beta amyloid in step (a) is body fluid.

5. The method according to claims 1 to 4, wherein the labeled beta amyloid added in step (b) is a beta amyloid which is recombinantly produced and labeled with at least one stable isotope.

6. The method according to claims 1 to 5, wherein the labeled beta amyloid added in step (b) is a beta amyloid which is synthetically produced and labeled with at least one stable isotope.

7. The method according to claims 1 to 6, wherein the beta amyloid added in step (b) is labeled with a stable isotope selected from the group comprising ¹⁵N, ¹³C, ¹⁸O and ²H.

8. The method according to claims 1, 4 to 7, wherein the beta amyloid in step (c) is isolated from body fluid by methods comprising protein chemistry and immunochemistry.

9. The method according to claims 2 to 3, 5 to 7, wherein the beta amyloid in step (c) is isolated from amyloid deposits by methods comprising dissolution with solubilizing agents.

10. The method according to claims 1 to 9, wherein the isolated beta amyloid in step (d) is prepared for analysis by mass spectrometry by methods comprising chemical reactions with flight enhancers, chemical fragmentation and enzymatic digestion.

11. The method according to claim 10, wherein the isolated beta amyloid in step (d) is prepared for analysis by mass spectrometry by enzymatic digestion with a protease selected from the group comprising endoproteinase Lys-C, trypsin, and endoproteinase Glu-C.

12. The method according to claims 1 to 11, wherein the prepared beta amyloid in step (e) is desalted before analysis by mass spectrometry.

13. The method according to claims 1 to 12, wherein the prepared beta amyloid in step (e) is analysed by MALDI-TOF mass spectrometry.

14. A method for the quantification of beta amyloid peptide comprising
(a) providing excised amyloid deposits from mammalian brain samples containing aggregated beta amyloid
(b) adding a defined amount of beta amyloid peptide labeled with a stable isotope
(c) dissolving the excised aggregated beta amyloid in the presence of the labeled beta amyloid
(d) digesting the dissolved beta amyloid with a protease
(e) analysing the digested beta amyloid peptide mixture by mass spectrometry, and
(f) determining the amount of beta amyloid that was present in the source of aggregated beta amyloid with the help of the base-line separation resulting from the presence of the natural and the stable isotopes in the beta amyloid.

15. Use of the methods according to claims 1 to 3, 5 to 7, 9 to 14, for the determination of the beta amyloid content in amyloid deposits containing aggregated beta amyloid obtained from tissue samples.

16. Use of the methods according to claims 1, 4 to 8, 10 to 13, for the determination of the beta amyloid content in body fluid containing soluble beta amyloid.

17. Use of the methods according to claims 1 to 14 for the quantification of amino terminal microheterogenous forms of beta amyloid.

18. Use of the methods according to claims 1 to 14 for the quantification of carboxy terminal microheterogenous forms of beta amyloid.

19. The method substantially as herein before described especially with reference to the foregoing Examples.
